# EUROPÄISCHE PATENTSCHRIFT

(11) **EP 1 808 136 B1**
(45) Veröffentlichungstag und Bekanntmachung des Hinweises auf die Patenterteilung: **10.12.2008**
(21) Anmeldenummer: 07000367.8
(22) Anmeldetag: 09.01.2007
(51) Int. Cl.: A61B 17/16, A61B 17/32

(54) **Chirurgische Kupplungsvorrichtung**
Surgical coupling device
Mécanisme d'embrayage chirurgicale

(30) Priorität: 12.01.2006 DE 102006001677
(43) Veröffentlichungstag der Anmeldung: 18.07.2007
(73) Patentinhaber: Gebr. Brasseler GmbH & Co. KG, 32657 Lemgo (DE)
(72) Erfinder: Danger, Karl-Heinz, 32758 Detmold (DE)
(74) Vertreter: Weber, Joachim

(56) Entgegenhaltungen:
- EP-B1- 1 006 898
- US-A- 4 705 038
- US-B1- 6 520 976

## Beschreibung

Die Erfindung bezieht sich auf eine chirurgische Kupplungsvorrichtung zur lösbaren Verbindung eines Handstücks mit einem chirurgischen Instrument.

Chirurgische Instrumente, welche in ein Handstück eingesetzt werden, sind in unterschiedlichsten Ausgestaltungsformen bekannt. Die Handstücke sind üblicherweise mit einem Antrieb verbunden, so dass Werkzeuge oder Teilbereiche der chirurgischen Instrumente antreibbar, beispielsweise in Drehung, versetzbar sind. Derartige chirurgische Instrumente können beispielsweise zahnärztliche Bohrer oder Fräser sein, sie können auch als Shaver-Instrumente für die Arthroskopie ausgebildet sein.

Aus dem Stand der Technik ist es bekannt (siehe beispielsweise EP 1 006 898 B1), das Handstück mit einer zentrischen Bohrung oder Ausnehmung zu versehen, in welche ein Kupplungsbereich oder Ansatzbereich des chirurgischen Instruments eingesteckt wird. Dabei gelangen entsprechende Antriebskupplungen des Handstücks in Eingriff mit Kupplungsteilen des chirurgischen Instruments, so dass ein Drehantrieb realisiert wird. Das Einstecken und Verrasten des chirurgischen Instruments in dem Handstück erfolgt dadurch, dass das Handstück mit einer Verrastungsausnehmung versehen ist, mit welcher ein Rastelement des chirurgischen Instruments lösbar in Eingriff bringbar ist. Die Verrastungsausnehmung kann beispielsweise in Form einer Ringnut ausgebildet sein, es ist auch möglich, diese nur singulär vorzusehen. Das Rastelement des chirurgischen Instruments kann beispielsweise in Form eines Rasthakens, einer Rastklinke oder Ähnlichem ausgebildet sein. Beim Einstecken des chirurgischen Instruments in das Handstück kann somit eine Verrastung oder Kupplung erfolgen, die ein unbeabsichtigtes Ablösen des chirurgischen Instruments von dem Handstück verhindert. Um das chirurgische Instrument von dem Handstück zu entnehmen, ist beispielsweise eine Betätigungseinrichtung zu bedienen.

Die aus dem Stand der Technik bekannten Vorrichtungen sind vielfach kompliziert aufgebaut und entsprechend kostenintensiv und aufwendig in der Herstellung und unter operativen Einsatzbedingungen vielfach schlecht betätigbar.

Der Erfindung liegt die Aufgabe zugrunde, eine chirurgische Kupplungsvorrichtung der eingangs genannten Art zu schaffen, welche bei einfachem Aufbau und einfacher, kostengünstiger Herstellbarkeit sicher und einfach bedienbar ist.

Erfindungsgemäß wird die Aufgabe durch die Merkmalskombination des Hauptanspruchs gelöst, die Unteransprüche zeigen weitere vorteilhafte Ausgestaltungen der Erfindung.

Erfindungsgemäß ist somit vorgesehen, dass das Rastelement mittels eines Servoantriebs betätigbar ist. Dieser Servoantrieb kann erfindungsgemäß beispielsweise in Form eines Servomotors oder eines Elektromagneten (Solenoid) ausgebildet sein. Hierdurch ergibt sich der entscheidende Vorteil, dass der Benutzer des Handstücks zum Einsetzen bzw. Entnehmen des Instruments keine weitere Verriegelungs- oder Entriegelungskraft aufbringen muss. Dies ist insbesondere im chirurgischen Umfeld von Vorteil, da dort durch die OP-Handschuhe feinfühlige Betätigungen von Hebeln, Schiebeknöpfen, Rastern oder Ähnlichem vielfach nicht oder nur erschwert möglich sind.

Erfindungsgemäß ergibt sich somit eine einfache Handhabung, welche zusätzlich die Betriebssicherheit ganz erheblich steigert.

In günstiger Weiterbildung der Erfindung ist vorgesehen, dass der Servoantrieb manuell betätigbar ist. Er kann jedoch in alternativer Ausgestaltung der Erfindung auch mittels eines Sensors betätigt werden, wobei der Sensor eine automatische oder semi-automatische Betätigung des Servoantriebs ermöglicht. So ist es beispielsweise möglich, beim Einsetzen des Instruments in das Handstück das Rastelement dann, wenn das Instrument korrekt eingesetzt ist, automatisch in seine Verriegelungsposition zu bringen. Es ist dann lediglich zur Freigabe des Instruments erforderlich, den Servoantrieb zu betätigen, sei es manuell oder automatisch. Eine derartige automatische Betätigung des Servoantriebs kann auch mit einem Steuerungsprogramm für das Handstück bzw. das Instrument gekoppelt sein. Dieses kann den Antrieb ausschalten, wenn das Instrument gewechselt werden soll, wobei dann entsprechend das Rastelement automatisch in seine Freigabeposition gebracht wird, damit die Bedienungsperson das Instrument wechseln kann.

Erfindungsgemäß ergibt sich somit auch die Möglichkeit, das Instrument mittels eines Operationsroboters zu handhaben und entsprechend automatisch zu wechseln.

Es versteht sich, dass eine automatische und eine manuelle Betätigbarkeit des Servoantriebes gleichzeitig vorgesehen sein können. Bei einer manuellen Betätigbarkeit kann beispielsweise ein Taster oder Schalter vorgesehen sein, der durch einfaches Drücken das Rastelement öffnet bzw. schließt (in seine Verriegelungsposition bringt bzw. in seine Freigabeposition überführt).

Der Servoantrieb kann erfindungsgemäß einen Servomotor umfassen, der in dem Instrument oder in dem Handstück untergebracht sein kann. Es versteht sich, dass die Erfindung auch eine mechanische Umkehr umfassen kann. Erfindungsgemäß sind auch gemischte Bauweisen denkbar, bei welchen das Rastelement an dem Instrument angeordnet ist, dieses jedoch mittels eines Servoantriebs betätigt wird, der sich an dem Handstück befindet.

Anstelle eines Servomotors kann auch ein Elektromagnet vorgesehen sein, der, wie erläutert, das Rastelement zwischen den beiden Positionen (Verriegelungsposition und Freigabeposition) schaltet. Dabei ist es möglich, das Rastelement als Teil des Elektromagneten (Solenoid) auszubilden, so dass beispielsweise der Kern des Elektromagneten die Funktion des Rastelements übernimmt. Hierdurch kann sich eine kostengünstige und einfache Ausgestaltung ergeben.

Die Energieversorgung des Servoantriebs kann mittels einer Batterie erfolgen, die an dem Instrument angebracht ist. Es ist auch möglich, die Stromversorgung über das Handstück vorzunehmen, entweder direkt oder unter Zwischenschaltung einer Akkumulatorbatterie in dem Instrument. Durch die erfindungsgemäße Ausgestaltung ist es weiterhin möglich, das Rastelement im stromlosen Zustand des Servoantriebs entweder in der Verriegelungsposition oder in der Freigabeposition zu halten. Somit kann die erfindungsgemäße Ausgestaltung ein zusätzliches Maß an Betriebssicherheit umfassen, falls sich während des Betriebs eine Störung der Energieversorgung ergeben sollte.

In einer Weiterbildung der Erfindung ist es möglich, mehrere Instrumente in einem Instrumentenspender bereitzustellen. Somit kann die Bedienungsperson im Rahmen einer EinhandBedienung die einzelnen Instrumente aus dem Instrumentenspender entnehmen und mit dem Handstück koppeln bzw. die Instrumente wieder in den Instrumentenspender ablegen. Hieraus ergibt sich ein hohes Maß an Betriebssicherheit und Bedienungskomfort. Durch die Möglichkeit, den Servoantrieb mittels eines Sensors zu steuern, kann das Instrument freigegeben werden, sobald es vollständig in den Instrumentenspender eingelegt oder eingesteckt wurde. Analog kann bei vollständiger Aufnahme eines neuen Instruments dieses automatisch verriegelt werden, bevor es aus dem Instrumentenspender entnommen wird.

Durch die beschriebene Zuordnung ist es auch möglich, die für eine Operation richtige und vorbestimmte Abfolge einzelner Instrumente festzulegen. Hierdurch kann verhindert werden, dass der Operateur zur falschen Zeit das falsche Instrument in das Handstück einsetzen möchte. Weiterhin kann eine zusätzliche Qualitätskontrolle der einzelnen Instrumente erfolgen, so dass beispielsweise ein Wiedereinsetzen eines bereits benutzten, stumpfen Instruments verhindert werden kann. Somit können beispielsweise thermische Schäden am Gewebe des Patienten vermieden werden.

Es versteht sich, dass die manuelle Betätigbarkeit des Servoantriebs unterschiedliche Arten von Tastern, Schaltern, etc., umfasst, so dass auch Fußschalter oder ähnliches möglich sind, die beispielsweise mit weiteren Betätigungselementen, beispielsweise für den Antrieb des Instruments, gekoppelt sein können.

Im Folgenden wird die Erfindung anhand eines Ausführungsbeispiels in Verbindung mit der Zeichnung beschrieben. Dabei zeigen:
- Fig. 1: eine schematische Teil-Schnittansicht der erfindungsgemäßen Kupplungsvorrichtung im verriegelten Zustand, und
- Fig. 2: eine Ansicht, analog Fig. 1, im freigegebenen Zustand.

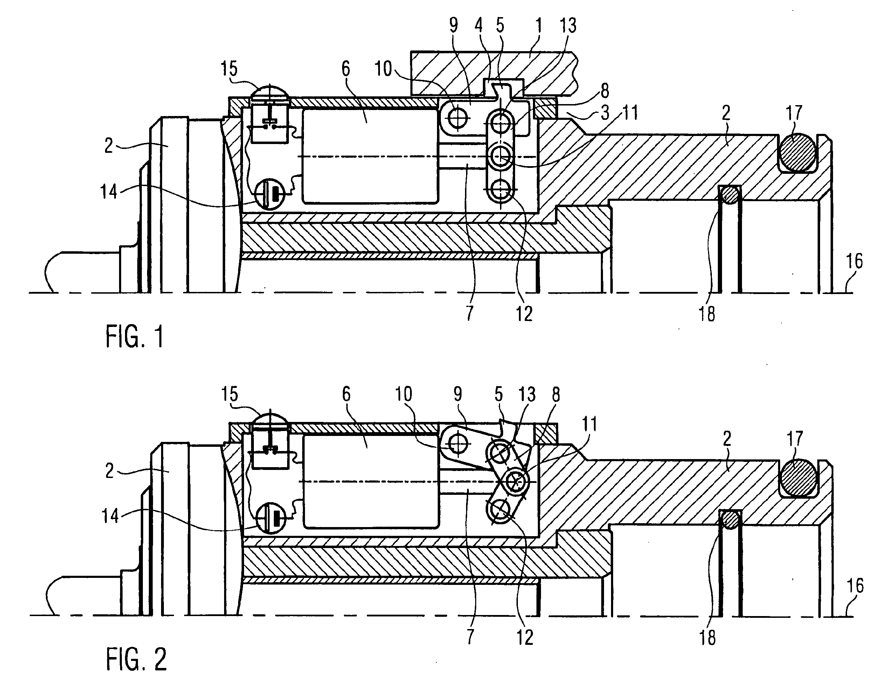

Die Fig. 1 zeigt in stark vereinfachter Darstellung einen Teil eines chirurgischen Handstücks 1, in welches ein chirurgisches Instrument 2 einsetzbar ist. Das Einsetzen des Instruments 2 erfolgt in eine Ausnehmung 3 des Handstücks 1. Das Handstück 1 ist mit einer Verrastungsausnehmung 4 versehen, welche als Ringnut oder als singuläre Ausnehmung ausgestaltet werden kann.

In die Verrastungsausnehmung 4 ist ein hakenförmiges Verrastungselement 5 einbringbar. Bei dem gezeigten Ausführungsbeispiel ist die Position des Rastelements 5 mittels eines Servoantriebs 6 wählbar. Der Servoantrieb 6 kann in Form eines Elektromotors oder eines Elektromagneten ausgebildet sein. Eine Betätigung des Rastelements 5 erfolgt über eine Stange 7, welche über einen Kniehebelmechanismus 8 mit einem Rasthebel 9 gekoppelt ist. Der Rasthebel 9 ist einstückig mit dem Rastelement 5 versehen und um eine ortsfeste Drehachse 10 schwenkbar. Am freien Ende der Stange 7 ist eine Drehachse 11 vorgesehen, mittels derer die beiden Hebel des Kniehebelmechanismus 8 schwenkbar mit der Stange 7 gekoppelt sind. Der untere Hebel des Kniehebelmechanismus 8 ist schwenkbar an einer ortsfesten Drehachse 12 gelagert, während der obere Hebel gelenkig an einer Drehachse 13 mit dem Rasthebel 9 verbunden ist.

Der Servoantrieb 6 ist mit einer Batterie 14 verbunden. Weiterhin ist ein Taster 15 vorgesehen, der manuell betätigbar ist.

Die Figuren zeigen weiterhin Dichtungen 17 und 18 sowie die Mittelachse 16 der Kupplungsvorrichtung.

Die Fig. 1 zeigt das Rastelement in der Verriegelungsposition. In dieser ist der Kniehebelmechanismus 8 durch Bewegung der Stange 7 nach links gespreizt. Hierdurch ergibt sich eine sichere Halterung des Rasthebels 5. Zum Lösen des Rasthebels 5, und um diesen in die in Fig. 2 gezeigte Freigabeposition zu bringen, wird die Stange 7 nach rechts verschoben. Hierdurch verschwenkt der Kniehebelmechanismus 8 den Rasthebel 9, so wie dies in Fig. 2 gezeigt ist.

### Bezugszeichenliste

- 1: Handstück
- 2: Chirurgisches Instrument
- 3: Ausnehmung
- 4: Verrastungsausnehmung
- 5: Rastelement
- 6: Servoantrieb
- 7: Stange
- 8: Kniehebelmechanismus
- 9: Rasthebel
- 10: Drehachse
- 11: Drehachse
- 12: Drehachse
- 13: Drehachse
- 14: Batterie
- 15: Taster
- 16: Mittelachse
- 17: Dichtung
- 18: Dichtung

## Patentansprüche

1. Chirurgische Kupplungsvorrichtung zur lösbaren Verbindung eines Handstücks (1) mit einem chirurgischen Instrument (2), wobei das Handstück (1) mit einer Ausnehmung (3) versehen ist, in welcher ein Kupplungsbereich des Instruments (2) lösbar einschiebbar ist, wobei an einer innenliegenden Wandung der Ausnehmung (3) eine Verrastungsausnehmung (4) ausgebildet ist, mit welcher ein Rastelement (5) lösbar in Eingriff bringbar ist, welches an dem Instrument (2) gelagert ist, **dadurch gekennzeichnet, dass** das Rastelement (5) mittels eines Servoantriebs (6) wahlweise in eine Verriegelungsposition und eine Freigabeposition bringbar ist.

2. Vorrichtung nach Anspruch 1, **dadurch gekennzeichnet, dass** der Servoantrieb (6) in Form eines Servomotors ausgebildet ist.

3. Vorrichtung nach Anspruch 1, **dadurch gekennzeichnet, dass** der Servoantrieb (6) in Form eines Elektromagneten ausgebildet ist.

4. Vorrichtung nach einem der Ansprüche 1 bis 3, **dadurch gekennzeichnet, dass** der Servoantrieb (6) manuell betätigbar ist.

5. Vorrichtung nach einem der Ansprüche 1 bis 3, **dadurch gekennzeichnet, dass** der Servoantrieb (6) mittels eines Sensors betätigbar ist.

6. Vorrichtung nach Anspruch 5, **dadurch gekennzeichnet, dass** der Sensor an dem Handstück (1) angeordnet ist.

7. Vorrichtung nach Anspruch 5, **dadurch gekennzeichnet, dass** der Sensor an dem Instrument (2) angeordnet ist.

8. Vorrichtung nach Anspruch 5, **dadurch gekennzeichnet, dass** der Sensor separat angeordnet ist.

9. Vorrichtung nach einem der Ansprüche 1 bis 3, **dadurch gekennzeichnet, dass** der Servoantrieb (6) automatisch, mit einer Steuerung des Handstücks (1) gekoppelt, betätigbar ist.

10. Vorrichtung nach einem der Ansprüche 1 bis 9, **dadurch gekennzeichnet, dass** das Rastelement (5) im stromlosen Zustand des Servoantriebs (6) in der Verriegelungsposition gehalten ist.

11. Vorrichtung nach einem der Ansprüche 1 bis 9, **dadurch gekennzeichnet, dass** das Rastelement (5) im stromlosen Zustand des Servoantriebs (6) in der Freigabeposition gehalten ist.

12. Chirurgisches Instrument mit einer Kupplungsvorrichtung nach einem der Ansprüche 1 bis 11

## Claims

1. Surgical coupling device for detachably connecting a tool holder (1) to a surgical instrument (2), the tool holder (1) being provided with a recess (3) in which a coupling portion of the instrument (2) is detachably insertable, wherein a locking recess (4) is formed in an inside wall of the recess (3), with which a locking member (5) may be engaged detachably, the locking member being supported at the instrument (2), **characterized in that** the locking member (5) may selectively be moved into a locking position and a release position by means of a servo drive (6).

2. The device of claim 1, **characterized in that** the servo drive (6) is formed as a servo motor.

3. The device of claim 1, **characterized in that** the servo drive (6) is formed as a solenoid.

4. The device of one of claims 1 to 3, **characterized in that** the servo drive (6) may be operated manually.

5. The device of one of claims 1 to 3, **characterized in that** the servo drive (6) may be operated by means of a sensor.

6. The device of claim 5, **characterized in that** the sensor is disposed at the tool holder (1).

7. The device of claim 5, **characterized in that** the sensor is disposed at the instrument (2).

8. The device of claim 5, **characterized in that** the sensor is arranged separately.

9. The device of one of claims 1 to 3, **characterized in that** the servo drive (6) may be operated automatically and coupled to a controller of the tool holder (1).

10. The device of one of claims 1 to 9, **characterized in that** the locking member (5) is maintained in the locking position in a power-off state of the servo drive (6).

11. The device of one of claims 1 to 9, **characterized in that** the locking member (5) is maintained in the release position in a power-off state of the servo drive (6).

12. A surgical instrument comprising a coupling device according to one of claims 1 to 11.

## Revendications

1. Dispositif de couplage chirurgical pour l'assemblage amovible d'une pièce à main (1) avec un instrument chirurgical (2), la pièce à main (1) étant munie d'un évidement (3), dans lequel peut être insérée de manière amovible une zone de couplage de l'instrument (2), sachant que sur une paroi intérieure de l'évidement (3) est ménagé un évidement de blocage (4), dans lequel peut entrer en prise de manière amovible un élément de blocage (5), qui est logé sur l'instrument (2), **caractérisé en ce que** l'élément de blocage (5) peut être amené au moyen d'un servomécanisme (6) au choix dans une position de verrouillage et une position de déblocage.

2. Dispositif selon la revendication 1, **caractérisé en ce que** le servomécanisme (6) est réalisé sous la forme d'un servomoteur.

3. Dispositif selon la revendication 1, **caractérisé en ce que** le servomécanisme (6) est réalisé sous la forme d'un électroaimant.

4. Dispositif selon l'une quelconque des revendications 1 à 3, **caractérisé en ce que** le servomécanisme (6) peut être actionné manuellement.

5. Dispositif selon l'une quelconque des revendications 1 à 3, **caractérisé en ce que** le servomécanisme (6) peut être actionné au moyen d'un capteur.

6. Dispositif selon la revendication 5, **caractérisé en ce que** le capteur est disposé sur la pièce à main (1).

7. Dispositif selon la revendication 5, **caractérisé en ce que** le capteur est disposé sur l'instrument (2).

8. Dispositif selon la revendication 5, **caractérisé en ce que** le capteur est disposé séparément.

9. Dispositif selon l'une quelconque des revendications 1 à 3, **caractérisé en ce que** le servomécanisme (6), couplé à une commande de la pièce à main (1), peut être actionné automatiquement.

10. Dispositif selon l'une quelconque des revendications 1 à 9, **caractérisé en ce que** l'élément de blocage (5) est maintenu dans la position de verrouillage lorsque le servomécanisme (6) est à l'état non sollicité par le courant.

11. Dispositif selon l'une quelconque des revendications 1 à 9, **caractérisé en ce que** l'élément de blocage (5) est maintenu dans la position de déblocage lorsque le servomécanisme (6) est à l'état sollicité par le courant.

12. Instrument chirurgical comportant un dispositif de couplage selon l'une quelconque des revendications 1 à 11.
